# EUROPÄISCHE PATENTSCHRIFT

(11) **EP 1 646 341 B1**
(45) Veröffentlichungstag und Bekanntmachung des Hinweises auf die Patenterteilung: **15.04.2009**
(21) Anmeldenummer: 04707934.8
(22) Anmeldetag: 04.02.2004
(51) Int. Cl.: A61F 2/46

(54) **EINSETZINSTRUMENT FÜR ZERVIKALPROTHESEN**
INSTRUMENT FOR INSERTING CERVICAL PROSTHESES
INSTRUMENT D'INSERTION POUR PROTHESES CERVICALES

(30) Priorität: 15.07.2003 US 619180
(43) Veröffentlichungstag der Anmeldung: 19.04.2006
(73) Patentinhaber: Cervitech, Inc., Rockaway, NJ 07866 (US)
(72) Erfinder: KELLER, Arnold, 23863 Kayhude (DE)
(74) Vertreter: Glawe, Delfs, Moll
(86) Internationale Anmeldenummer: PCT/EP2004/001028
(87) Internationale Veröffentlichungsnummer: WO 2005/007044

(56) Entgegenhaltungen:
- EP-A- 0 333 990
- WO-A-02/071986
- DE-U- 29 916 078
- FR-A- 2 795 945
- US-A- 6 159 215
- US-A1- 2003 105 467

## Beschreibung

Die Erfindung betrifft ein Einsetzinstrument für eine mehrteilige Zwischenwirbelendoprothese, die zwei Abschlußplatten und einen dazwischen angeordneten Gleitkern umfaßt, mit einem Griffteil, Greifgliedern, die die Abschlußplatten zwischen sich halten, und einem Kraftaufnahmeteil zur Ausübung einer Einsetzkraft auf die Zwischenwirbelendoprothese.

Zum Einsetzen von Zwischenwirbelprothesen ist ein Einsetzinstrument bekannt (EP-A-1 306 064), das an seinem vorderen Ende zwei Prothesenhalterungen zur Aufnahme von jeweils einer Prothesenplatte aufweist, die aus zwei starr miteinander verbundenen Greifgliedern bestehen, die die Platten durch Reibkraft zwischen sich halten. Für sehr kleine Implantate, wie sie-im Bereich der Halswirbelsäule verwendet werden und die sehr genau plaziert werden müssen, kann dies zu unsicher sein.

Ein Einsetzinstrument gemäß dem Oberbegriff des Anspruchs 1 wird in EP-A-0333990 beschrieben.

Der Erfindung liegt die Aufgabe zugrunde, ein verbessertes Instrument für die Implantation von Zwischenwirbelprothesen zu schaffen, das insbesondere an die Bedürfnisse bei der Implantation unter beengten Verhältnisse angepaßt ist, wie sie vor allem im Bereich der Halswirbelsäule auftreten.

Die erfindungsgemäße Lösung liegt in einem Einsetzinstrument mit den Merkmalen des Anspruchs 1. Vorteilhafte Weiterbildungen sind Gegenstand der Unteransprüche.

Erfindungsgemäß ist bei einem Einsetzinstrument für eine mehrteilige Zwischenwirbelendoprothese, insbesondere eine Zervikalprothese, die zwei Abschlußplatten und einen dazwischen angeordneten Gleitkern umfaßt, mit einem Griffteil, Greifgliedern, die die Abschlußplatten zwischen sich halten, und einem Kraftaufnahmeteil zur Ausübung einer Einsetzkraft auf die Zwischenwirbelendoprothese, vorgesehen, daß die Greifglieder über ein Gelenk zueinander hin- und herbeweglich geführt und gegen die Zwischenwirbelendoprothese spannbar sind, in Spannrichtung weisende Vorsprünge oder Ausnehmungen angeordnet und zum formschlüssigen Halten der Zwischenwirbelendoprothese an den Greifgliedern ausgebildet sind, und ein in Längsrichtung geführter Block mit einer Anschlagfläche vorgesehen ist, der zur Zwischenwirbelendoprothese mittels einer Betätigungseinrichtung bewegbar ist und in seiner vorderen Position die Zwischenwirbelendoprothese gegen die Vorsprünge oder Ausnehmungen festlegt. Die gelenkig miteinander verbundenen Greifglieder bewegen sich beim Schließen des zangenförmigen Einsetzinstruments aufeinander zu und greifen mit ihren Vorsprüngen (oder Ausnehmungen) formschlüssig in entsprechende Vertiefungen (oder Erhebungen) der Zwischenwirbelendoprothese ein und spannen sie damit in eine Richtung quer zur Längsachse des Einsetzinstruments. Der längsbeweglich geführte Block kann zur Zwischenwirbelendoprothese hin bewegt werden, bis seine Anschlagfläche an der Zwischenwirbelendoprothese anliegt und sie gegen die Vorsprünge (oder Ausnehmungen) festlegt. Damit ist die Zwischenwirbelendoprothese auch in Längsrichtung gespannt. Sie ist somit spielfrei und positionsgenau von dem Einsetzinstrument gehalten. Dank des fest an der Zischenwirbelendoprothese anliegenden Blocks können dabei auch große Kräfte, wie sie beim Einschlagen der Zwischenwirbelendoprothese auftreten, sicher übertragen werden. Da die großen Kräfte durch den Block und seine Anschlagfläche übertragen werden, brauchen die Vorsprünge (oder Ausnehmungen) diese Kräfte nicht aufzunehmen. Sie können schwächer dimensioniert und so fein ausgeführt sein, wie es für eine genaue Positionierung gewünscht ist, ohne auf die hohe Kraftbelastung beim Einschlagen Rücksicht nehmen zu müssen. Außerdem verhindert der Block durch seine Anlage an die Zwischenwirbelendoprothese, daß diese sich unbeabsichtigt verdreht oder ihre einzelnen Elemente aufklappen. Dank der Erfindung kann die Zwischenwirbelendoprothese somit positionsgenau, sicher und auf einfache Weise an dem Einsetzinstrument gehalten und eingesetzt-werden.

### Nachfolgend seien einige Begriffe erläutert:

Unter der Längsachse der Zange wird die Mittellinie verstanden, welche die Winkelhalbierende zwischen den Griffteilen der Zangenhälften und den Backenteilen der Zangenhälften ist.

Unter Spannrichtung wird die Richtung verstanden, in welche sich die Greifglieder aufeinander zu bewegen. Die entgegengesetzte Richtung ist die Spreizrichtung. Diese Richtungen sind generell etwa quer zur Längsachse des Einsetzinstruments.

Unter formschlüssigen Halten wird verstanden, daß die Vorsprünge in entsprechend ausgebildete Aufnahmeöffnungen der Zwischenwirbelendoprothese oder umgekehrt eingreifen. In Richtung der Längsachse gesehen greifen die Vorsprünge in einen Hinterschnitt.

Vorzugsweise ist das Einsetzinstrument als Zange ausgebildet, deren Backenteile die Greifglieder bilden. Das ermöglicht eine raumsparende Konstruktion und einfache Handhabung, was insbesondere bei den beengten Verhältnissen im Halswirbelbereich von Vorteil ist.

Zur leichteren Verwendung weist die Betätigungseinrichtung eine Stange mit einer im hinteren Bereich des Griffteils angeordneten Handhabe auf. Diese ermöglicht es dem Operateur, die Betätigungseinrichtung ohne umständliches Umgreifen zu verwenden. Dies ist insbesondere beim Abnehmen des Einsetzinstruments aufgrund der beengten Platzverhältnisse bei Zervikalprothesen nach dem Einbringen der Zwischenwirbelendoprothese von Bedeutung, wenn der Block zurückbewegt werden muß. Zweckmäßigerweise ist dazu die Stange, mit einem Schraubgewinde versehen und in einem instrumentenfest, vorzugsweise im Gelenk angeordneten Gegengewinde geführt. So kann durch Drehung in eine Richtung der Block gegen die Zwischenwirbelendoprothese geführt werden und sie damit festlegen, während durch Drehung in die entgegengesetzte Richtung der Block wegbewegt wird und die Zwischenwirbelendoprothese zum Abnehmen des Einsatzinstruments freigibt. Die Schraubeinrichtung hat ferner den Vorzug, selbsthemmend zu sein, so daß eine gesonderte Sicherungseinrichtung zum Schutz gegenüber unbeabsichtigter Verstellung nicht.erforderlich ist. Eine Schraubeinrichtung ist aber nicht unbedingt erforderlich, es können auch andere vorzugsweise selbsthemmende Betätigungseinrichtungen vorgesehen sein.

Eine besonders zweckmäßige Bauweise ist es, die Betätigungseinrichtung durch das Gelenk zu führen. Dies ist nicht nur eine besonders raumsparende Ausführung, sondern gewährleistet auch eine mittennahe Anordnung. Diese Anordnung stellt sicher, daß das Einsetzinstrument auch unter hoher Kraftbelastung beim Schlagen nicht zur Seite hin ausweicht. Dadurch wird eine hohe Positionierungsgenauigkeit beim Einsetzen der Zwischenwirbelendoprothese erreicht.

Es ist zweckmäßig, wenn die Betätigungseinrichtung an ihrem griffseitigen Ende einen Schlagkopf aufweist. Damit kann über die Betätigungseinrichtung und dem Block direkt auf die Zwischenwirbelendoprothese eingewirkt werden, um sie an ihren Implantationsort zu bringen. Zweckmäßigerweise ist dazu die Handhabe selbst als Schlagkopf ausgebildet. Dies ermöglicht eine raumsparende Konstruktion, was insbesondere bei den beengten Verhältnissen im Bereich der Halswirbelsäule von großem Wert ist.

Um ein versehentliches Aufspringen des Einsetzinstruments auch unter hoher Kraftbelastung zu vermeiden, ist zweckmäßigerweise eine Rasteinrichtung zum Sichern der Griffteile in zusammengedrückter Position vorgesehen, die eine Führung für die Betätigungseinrichtung aufweist. Das verhindert insbesondere bei weit hinten angeordnetem Schlagkopf ein Ausknicken der Betätigungseinrichtung unter hohen Belastungen. Die Rast-einrichtung kann in an sich bekannter Weise am hinteren Ende der Griffteile vorgesehen sein. Wichtig ist, daß sie hinreichend kräftig dimensioniert ist, so daß sie den beim Schlagen auftretenden Belastungen standhält, sich aber zum Abnehmen des Instruments leicht lösen läßt.

Bei einer zweckmäßigen Ausführungsform sind die Vorsprünge oder Ausnehmungen an Backeneinsätzen angeordnet, die auswechselbar an den Backenteilen befestigt sind. Dies ermöglicht bei Bedarf ein leichtes Auswechseln der Backeneinsätze mit den daran angeordneten Vorsprüngen oder Ausnehmungen, um das Einsetzinstrument an andere Typen oder Größen von Zwischenwirbelendoprothesen anzupassen.

Die Erfindung wird nachfolgend unter Bezugnahme auf die Zeichnung erläutert, in der ein vorteilhaftes Ausführungsbeispiel dargestellt ist. Es zeigen:
- Fig. 1: eine Gesamtansicht des erfindungsgemäßen Einsetzinstruments von seiner Oberseite mit einer Zwischenwirbelendoprothese;
- Fig. 2: eine Gesamtansicht des erfindungsgemäßen Einsetzinstruments von seiner Unterseite;
- Fig. 3: eine vergrößerte Teilansicht eines Backenteils des Einsetzinstruments in einem Längsachsenschnitt;
- Fig. 4: eine Teilansicht des eines anderen Backeneinsatz; und
- Fig. 5: eine Teilansicht des Einsetzinstruments mit daran angeordneter Zwischenwirbelendoprothese.

Das in den Figuren dargestellte Ausführungsbeispiel für ein erfindungsgemäßes Einsetzinstrument ist eine Zange, die in seiner Gesamtheit mit dem Bezugszeichen 1 versehen ist. Sie dient zum Einsetzen von Zervikalprothesen 9 in den Zwischenraum zweier benachbarter Wirbelkörper der Halswirbelsäule (nicht dargestellt).

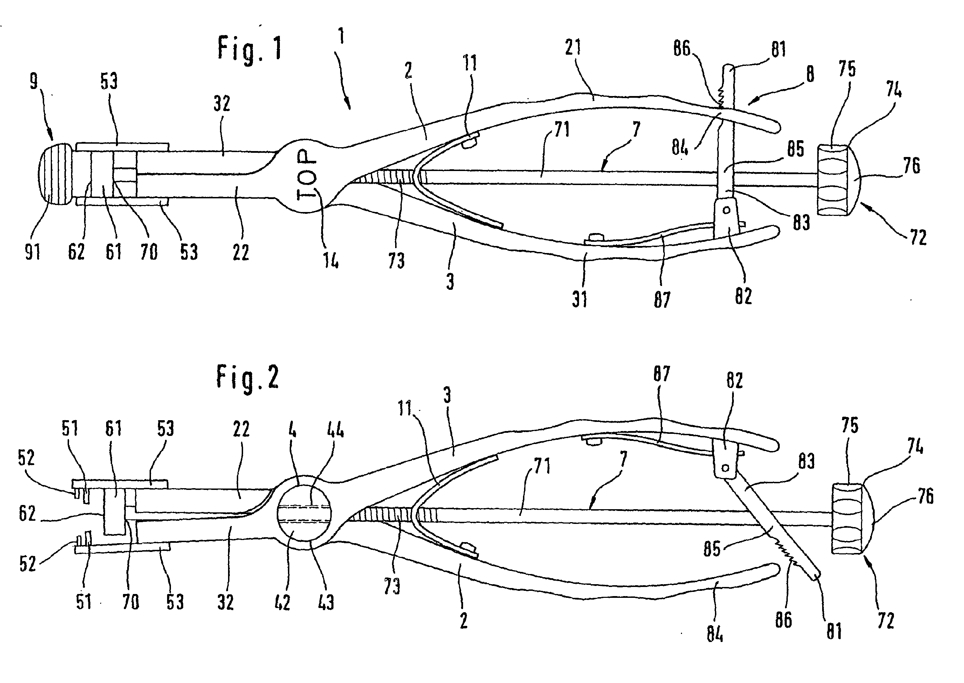

Die Zange 1 ist aus zwei Zangenhälften 2, 3 gebildet, die über ein Schwenkgelenk 4 beweglich miteinander verbunden sind. Die Zangenhälften 2, 3 weisen in ihrem hinteren Bereich jeweils ein Griffteil 21, 31 und in ihrem vorderen Bereich jeweils ein Backenteil 22, 32 auf. In dem Übergang zwischen den Griffteilen 21, 31 und den Backenteilen 22, 32 ist das Schwenkgelenk 4 angeordnet. Es ist gebildet durch einen Zapfen 42 an der Zangenhälfte 2 (in Fig. 1 erstreckt er sich aus der Zeichenebene nach oben heraus), der in einer passenden Öffnung 43 in dem mittleren Bereich der anderen Zangenhälfte 3 gelagert ist. Der Lagerzapfen 42 weist eine Durchgangsbohrung 44 auf, die von dem griffseitigen Bereich der Zangenhälften 2, 3 zu dem backenseitigen Bereich läuft. Sie wird später noch näher erläutert werden. Das Schwenklager 4 erlaubt es, die Griffteile 21, 31 der Zangenhälften 2, 3 aufeinander zu zu bewegen, damit sich die Backenteile 22, 32 schließen und umgekehrt.

Die Backenteile 22, 32 fungieren als Greifglieder. Sie weisen im vorderen Bereich an ihren einander zugewandten Innenseiten jeweils zwei in Spannrichtung 12 weisende Vorsprünge 51, 52 auf. Diese Vorsprünge sind nicht unmittelbar an den Backenteilen 22, 32, sondern an Backeneinsätzen 53 angeordnet, die mittels einer Schraube (nicht dargestellt) auswechselbar an den Außenseiten der Backenteile 22, 32 in einer entsprechenden Aufnahme befestigt sind. Jeder Backeneinsatz 53 weist je einen Vorsprung 51 und einen Vorsprung 52 auf. Der Vorsprung 51 ist stiftartig geformt und befindet sich im oberen Bereich, während der Vorsprung 52 plättchenartig geformt ist und sich im unteren Bereich des Backeneinsatzes 53 befindet. Die Abmessungen und die Anordnung der Vorsprünge 51, 52 sind auf entsprechende Aufnahmeöffnungen an den aufzunehmenden Zervikalprothesen 9 angepaßt. Dies wird später noch näher erläutert werden.

An dem Backenteil 22 ist eine Führungsschiene 60 angeordnet, die einen Block 61 längsverschieblich an der Zangenhälfte 2 in Vor- und Rückwärtsrichtung beweglich hält. Die Führungsschiene 60 ist als ein Langloch in dem Backeneinsatz 53 des Backenteils 32 ausgebildet. Eine seitlich in dem Block 61 angeordnete Madenschraube greift in das die Führungsschiene 60 bildende Langloch ein und führt den Block in Längsrichtung. Anstelle des Langlochs können auch andere Führungselemente vorgesehen sein, die eine in Längsrichtung vor- und zurückbewegliche Führung des Blocks 61 erlauben, beispielsweise eine Schwalbenschwanzführung. Der Block 61 ist an seinem vorderen Ende mit einer Anschlagfläche 62 versehen, die zum Zusammenwirken mit der Zervikalprothese 9 ausgebildet ist.

An dem Block 61 greift eine Betätigungseinrichtung 7 an, die sich von dem hinteren Bereich des Blocks 61 durch die Durchgangsbohrung 44 in den Bereich zwischen den Griffteilen 21, 31 erstreckt. Die Betätigungseinrichtung 7 umfaßt ein Kopplungselement 70 mit dem Block 61, das in dem dargestellten Ausführungsbeispiel ein zur Übertragung von Schubkräften geeignetes Wirbellager ist, ferner eine Stange 71 und eine Handhabe 72 zur Betätigung. Im vorderen Bereich der Stange 71 ist ein Außengewinde 73 vorgesehen, das mit einem komplementären Innengewinde (nicht dargestellt) in der Durchgangsbohrung 44 des Zapfens 42 als instrumentenfeste Führung zusammenwirkt. Damit ist es ermöglicht, durch Drehen der Handhabe 72 die Stange 71 und damit über das Kopplungselement 70 den Block 61 entlang der Führungsschiene 60 vor- und zurückzubewegen. Die Handhabe 72 ist als Drehknopf ausgebildet, der an seiner äußeren Mantelfläche 74 zur Bereitstellung einer guten Greifmöglichkeit für den Operateur eine geeignete Oberflächengestaltung, wie eine Grobriffelung 75, aufweist.

Das hintere Ende der Handhabe 72 ist mit einer konvexen Wölbung 76 versehen. Sie dient als Schlagkopf für die Betätigungseinrichtung 7. Sie überträgt Impulse von auf die Wölbung 76 des Schlagkopfes einwirkenden Schlägen über die Stange 71 der Betätigungseinrichtung 7, das schubfeste Wirbellager 70 und den Block 61 auf dessen Anschlagfläche 62.

Im hinteren Bereich der Zange 1 ist eine Rasteinrichtung 8 für die Griffteile 21, 31 vorgesehen. Sie umfaßt ein schwenkbewegliches Verrastungselement 83 und eine Rastklinke 84 (die jeweils gegenüberliegend an den Griffteilen 21, 31 angeordnet sind), eine Löseeinrichtung 81, einen Fuß 82 und eine Feder 87. Das hintere Ende des Griffteils 21 ist als Gabel ausgeführt, wobei die Rastklinke 84 durch eine Anschrägung des Fußpunkt der Gabel gebildet ist. Das Verrastungselement 83 ist durch den Fuß 82 in der durch die Griffteile 21, 31 aufgespannten Ebene gelagert. Die Feder 87 ist als Blattfeder ausgebildet und wirkt auf das in dem Fuß 82 gelagerte Ende des Verrastungselements 83 so ein, daß es nach vorne zur Rastklinke 84 gedrückt wird. Ausgehend von dem Fuß 82 weist das Verrastungselement 83 einen breiten Bereich und einen schmalen Bereich auf. In seinem schmalen Bereich weist das Verrastungselement 83 an seiner Vorderseite eine Verzahnung 86 auf, in welche im geschlossenen Zustand der Zange 1 die Rastklinke 84 greift und verrastet, so daß die Griffteile 21, 31 sich nicht voneinander wegbewegen können und damit das Einsetzinstrument 1 gegenüber einem versehentlichen Aufspringen gesichert ist. Dadurch können auch erhebliche Belastungen, wie bspw. Hammerschläge auf die Wölbung 76, auf der Zange 1 ausgeübt werden, ohne daß ein versehentliches Öffnen zu befürchten ist und ohne daß der Operateur die Griffteile 21, 31 mit Handkraft gegenüber einem unerwünschten Öffnen zu sichern braucht. Zum Öffnen der Zange 1 nach erfolgter Implantation wird durch Druck nach hinten auf das Auslöseelement 81 das Verrastungselement 83 nach hinten geschwenkt, wodurch die Rastklinke 84 von dem Verrastungselement 83 freikommt und somit die Griffteile 21, 31 sich unter der Wirkung der Feder 11 auseinander bewegen. Im geöffneten Zustand der Zange 1 ist das Verrastungselement 83 gegen die Kraft der Feder 87 nach hinten geschwenkt. In dem breiten Bereich des Verrastungselements 83 ist eine als Langloch ausgebildete Führung 85 vorgesehen, die dazu dient, die Stange 71 auch bei geöffneter Zange 1 in einer definierten Position in der Längsachse 10 zu halten und um ein Ausknicken der Stange 71 auch unter hoher Schlagbelastung zu vermeiden.

An dem Griffteil 31 ist ferner eine Blattfeder 11 befestigt, die um die Stange 71 herum zu dem anderen Griffteil 21 geführt ist. Bei geschlossener Zange 1 steht diese Blattfeder 11 unter Spannung und bewirkt, daß das Einsetzinstrument 1 nach dem Lösen der Rastelemente 82, 83 selbsttätig öffnet, um ein Abnehmen zu ermöglichen.

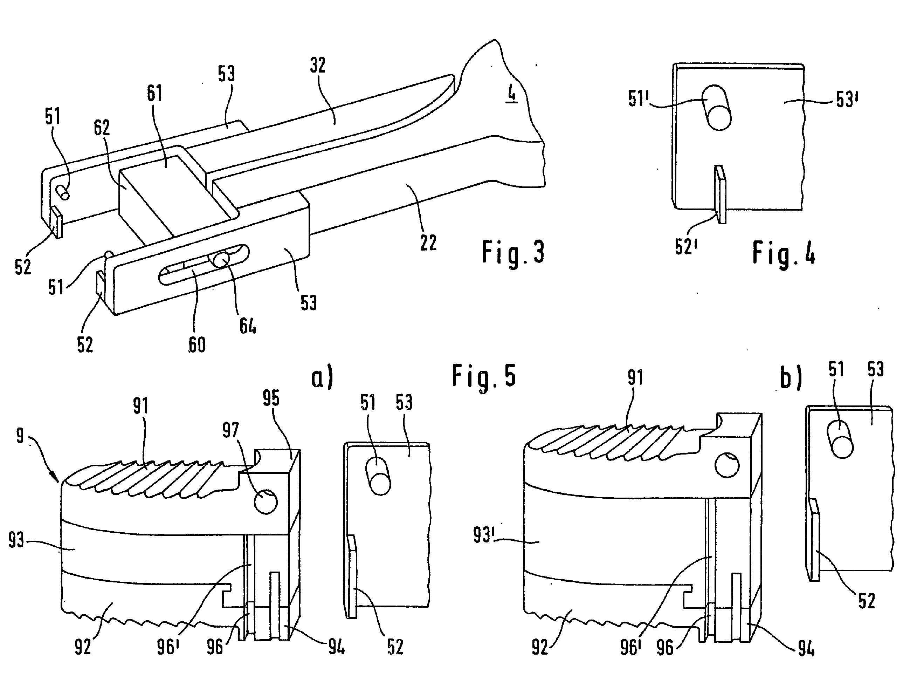

Nachfolgend soll unter Bezugnahme auf Fig. 3 und 5 das Zusammenwirken mit der Zervikalprothese 9 beschrieben werden. Die Zervikalprothese 9 besteht aus einer oberen Abschlußplatte 91 und einer unteren Abschlußplatte 92 mit einem dazwischen angeordneten Gelenk 93. Die zervikalprothese 9 ist zur Implantation in dem Zwischenraum zweier benachbarter Wirbel der Halswirbelsäule eines Menschen vorgesehen. Dabei wird die obere Abschlußplatte 91 an dem kranialen Wirbel und die untere Abschlußplatte 92 an dem kaudalen Wirbel befestigt. Um die Zervikalprothese 9 sicher an der Zange 1 zum Einführen in den Zwischenwirbelraum anzuordnen, weisen die obere und die untere Abschlußplatte 91, 92 an ihren seitlichen Flanken im Bereich ihres vorderen Flansches 94, 95 Aufnahmeöffnungen auf. Dabei ist die Aufnahmeöffnung an der oberen Abschlußplatte 91 als Bohrung 97 mit einer zusätzlichen Senkung ausgeführt. Die Aufnahmeöffnung an der unteren Abschlußplatte 92 ist als ein Schlitz 96 ausgeführt. Der Gleitkern 93 ist in seinem flanschseitigen Bereich ebenfalls mit einem Schlitz 96' versehen, der so angeordnet ist, daß er mit dem Schlitz 96 der unteren Abschlußplatte 92 fluchtet. Damit ergeben die Schlitze 96, 96' eine durchgehende Rinne.

Zum Aufnehmen der Zervikalprothese 9 mit der Zange 1 wird die Zervikalprothese 9 in dem Bereich zwischen die Backenteile 22, 32 gebracht und die Zange 1 geschlossen, wodurch sich die Backenteile 22, 32 aufeinander zu bewegen. Dabei greifen die Vorsprünge 51, 52 in die entsprechenden Aufnahmeöffnungen der beiden Abschlußplatten 91, 92 ein, wobei die Stifte 51 in die Bohrung 97 und die Plättchen 52 in die Schlitze 96, 96' eingreifen. Dadurch wird die Zervikalprothese 9 in Spannrichtung spielfrei an der Zange 1 gehalten: Die unterschiedliche Ausführung der Vorsprünge 51, 52 und der Aufnahmeöffnungen in Gestalt der Bohrungen 97 und Schlitze 96 stellt sicher, daß die Zervikalprothese 9 nur mit der richtigen Orientierung an der Zange 1 gehalten werden kann. Ist die Zange 1 wie in dem dargestellten Ausführungsbeispiel auch zusätzlich mit einer Markierung 14 für die Oberseite versehen, so sind Fehlimplantationen aufgrund verkehrter Orientierung der Zervikalprothese 9 praktisch ausgeschlossen. Nachdem auf diese Weise die Zervikalprothese 9 an der Zange 1 in der richtigen Orientierung aufgenommen wurde, kann mittels der Betätigungseinrichtung 7 durch Drehen der Handhabe 72 die Stange 71 nach vorne bewegt werden, so daß der Block 61 mit seiner Anschlagsfläche 62 von hinten an den Flansch 94, 95 der Zervikalprothese 9 zur Anlage kommt. Dabei spannt der Block 61 die Zervikalprothese 9 gegen die Vorsprünge 51, 52 und richtet so die Zervikalprothese 9 in eine definierte Position aus. Eventuell vorhandenes Spiel in Längsachsenrichtung zwischen den Vorsprüngen 51, 52 und den Bohrungen 97 und den Schlitzen 96 wird auf diese Weise herausgedrückt. Die Zervikalprothese 9 ist damit sicher und positionsgenau an der Zange 1 gehalten. Außerdem verhindert das Anliegen des Blocks 61 an den Flanschen 93, 94 der beiden Abschlußplatten 91, 92, daß sich die beiden Abschlußplatten 91, 92 an ihrem vorderen Ende voneinander wegbewegen. Dadurch wird ein Aufklappen der Zervikalprothese 9, das ein erfolgreiches Einbringen in den Zwischenwirbelraum verhindern würde, ausgeschlossen.

Weiter ist es ermöglicht, Zervikalprothesen mit anderer Höhe ohne Veränderungen an der Zange 1 zu implantieren. In Fig. 3b ist eine solche Zervikalprothese 9' dargestellt, die einen dickeren Gleitkern 93' aufweist. Er ist (ebenso wie der Gleitkern 93) mit einem Schlitz 96" versehen, der mit dem Schlitz 96 der unteren Abschlußplatte 92 fluchtet. Diese Gestaltung der Aufnahmeöffnung an der unteren Abschlußplatte 92 als Schlitz 96 und seine Fortsetzung als Schlitz 96" in dem Gleitkern 93' ermöglichen es, die dickere Zervikalprothese 9' mit derselben Zange 1 mit unveränderter Anordnung der Vorsprünge 51, 52 zu greifen und sicher zu halten. Die Positionierungsgenauigkeit ist dabei durch die stiftartigen Vorsprünge 51, die in die Bohrungen 97 eingreifen, gesichert.

Bei Bedarf können aber auch andere Backeneinsätze 53' vorgesehen sein, die eine andere Anordnung der Vorsprünge 51', 52' aufweisen, wie sie in Fig. 4 dargestellt ist. Damit kann die Zange 1 an andere Zwischenwirbelendoprothesen angepaßt werden, z. B. an besonders kleine zur Therapie von Kindern.

Der Block 61 stellt mit seiner Anschlagfläche 62 eine hinreichend groß dimensionierte Kraftübertragungsfläche zur Verfügung, um die auf die Wölbung 76 als Schlagkopf aufgebrachten Impulse auf die Zervikalprothese 9 zu übertragen. Das hat den großen Vorteil, daß die im Interesse einer genauen Positionierung feingliedrig ausgeführten Vorsprünge 51, 52 nicht die Schlagkräfte übertragen müssen, so daß die Gefahr eines Verbiegens oder gar Abbrechens der Vorsprünge 51, 52 aufgrund Überbelastung auch bei hoher Schlagbelastung dank des die Kraftübertragung übernehmenden Blocks 61 mit seiner Anschlagfläche 62 ausgeschlossen ist.

Die erfindungsgemäße Zange 1 ermöglicht eine verwechslungssichere und positionsgenaue Anordnung der Zervikalprothese 9 an der Zange 1, wobei ein unerwünschtes Aufklappen der Zervikalprothese 9 verhindert wird. Ferner ermöglicht sie dank des Blocks 61 mit der Anschlagfläche 62 auch bei kleindimensionierten Zangen 1 die Übertragung hoher Kräfte. Damit ist ein sicheres Implantieren der Prothese gewährleistet. Die kleine Dimensionierung der Zange 1 hat ferner den Vorteil, daß sie dem Operateur gute Sicht- und Zugangsbedingungen auf den Implantationsort gibt.

## Patentansprüche

1. Einsetzinstrument für eine mehrteilige Zwischenwirbelendoprothese (9), die zwei Abschlußplatten (91, 92) und einen dazwischen angeordneten Gleitkern (93) umfaßt, mit einem Griffteil (21, 31), Greifgliedern, die die Abschlußplatten zwischen sich halten, und einem Kraftaufnahmeteil zur Ausübung einer Einsetzkraft auf die Zwischenwirbelendoprothese (9),
**dadurch gekennzeichnet, daß**
die Greifglieder über ein Gelenk (4) zueinander hin- und herbeweglich geführt und gegen die Zwischenwirbelendoprothese (9) spannbar sind, in Spannrichtung (12) weisende Vorsprünge (51, 52) oder Ausnehmungen zum formschlüssigen Halten der Zwischenwirbelendoprothese (9) an den Greifgliedern ausgebildet sind, und ein in Längsachsenrichtung (10) geführter Block (61) mit einer Anschlagfläche (62) vorgesehen ist, der zur Anlage an die Zwischenwirbelendoprothese (9) mittels einer Betätigungseinrichtung (7) bewegbar ist und in seiner vorderen Position die Zwischenwirbelendoprothese (9) gegen die Vorsprünge (51, 52) oder Ausnehmungen festlegt.

2. Einsetzinstrument nach Anspruch 1,
**dadurch gekennzeichnet, daß**
das Einsetzinstrument als Zange (1) ausgebildet ist,
deren Backenteile (22, 32) die Greifteile bilden.

3. Einsetzinstrument nach Anspruch 1 oder 2,
**dadurch gekennzeichnet, daß**
die Betätigungseinrichtung (7) eine Stange (71) mit einer im hinteren Bereich des Griffteils (21) angeordneten Handhabe (72) ist.

4. Einsetzinstrument nach Anspruch 3,
**dadurch gekennzeichnet, daß**
die Stange (71) mit einem Schraubgewinde (73) versehen ist und in einem instrumentenfest, vorzugsweise im Gelenk (4) angeordneten Gegengewinde geführt ist.

5. Einsetzinstrument nach Anspruch 3 oder 4,
**dadurch gekennzeichnet, daß**
die Handhabe (72) als Schlagkopf (76) ausgebildet ist.

6. Einsetzinstrument nach einem der Ansprüche 2 bis 5,
**dadurch gekennzeichnet, daß**
die Betätigungseinrichtung (7) durch das Gelenk (4) geführt ist.

7. Einsetzinstrument nach einem der Ansprüche 1 bis 6,
**dadurch gekennzeichnet, daß**
eine Rasteinrichtung (8) zum Sichern der Griffteile (21, 31) in zusammengedrückter Position vorgesehen ist, die eine Führung (85) für die Betätigungseinrichtung (7) aufweist.

8. Einsetzinstrument nach einem der vorhergehenden Ansprüche,
**dadurch gekennzeichnet, daß**
die Vorsprünge (51, 52) an Backeneinsätzen (53) angeordnet sind, die auswechselbar an den Backenteilen (22, 32) befestigt sind.

## Claims

1. Insertion instrument for a multi-part intervertebral endoprosthesis (9) which comprises two closure plates (91, 92) and a sliding core (93) arranged between these, said insertion instrument having a handgrip part (21, 31), gripping members which hold the closure plates between them, and a force-receiving part for applying an insertion force to the intervertebral endoprosthesis (9),
**characterized in that**
the gripping members are guided movably toward and away from one another via a hinge (4) and are able to be tensioned against the intervertebral endoprosthesis (9), projections (51, 52) pointing in the tensioning direction (12) or recesses for holding the intervertebral endoprosthesis (9) with a form-fit are formed on the gripping members, and a block (61) guided in the longitudinal axis direction (10) and with an abutment surface (62) is provided which can be moved by means of an actuating device (7) so as to bear on the intervertebral endoprosthesis (9) and, in its forward position, secures the intervertebral endoprosthesis (9) against the projections (51 52) or recesses.

2. Insertion instrument according to Claim 1,
**characterized in that**
the insertion instrument is designed as a forceps (1), whose jaw parts (22, 32) form the gripping parts.

3. Insertion instrument according to Claim 1 or 2,
**characterized in that**
the actuating device (7) is a rod (71) with a handle (72) arranged in the rear area of the handgrip part (21).

4. Insertion instrument according to Claim 3,
**characterized in that**
the rod (71) is provided with a screw thread (73) and is guided in a counterthread which is fixed on the instrument and arranged preferably in the hinge (4).

5. Insertion instrument according to Claim 3 or 4,
**characterized in that**
the handle (72) is designed as a strike head (76).

6. Insertion instrument according to one of Claims 2 to 5,
**characterized in that**
the actuating device (7) is guided through the hinge (4).

7. Insertion instrument according to one of Claims 1 to 6,
**characterized in that**
a locking device (8) is provided for securing the handgrip parts (21, 31) in the position when pressed together, said locking device (8) having a guide (85) for the actuating device (7).

8. Insertion instrument according to one of the preceding claims,
**characterized in that**
the projections (51, 52) are arranged on jaw inserts (53) which are fastened releasably on the jaw parts (22, 32).

## Revendications

1. Instrument d'insertion pour une endoprothèse intervertébrale en plusieurs parties (9), qui comprend deux plaques de fermeture (91, 92) et un noyau de glissement (93) disposé entre celles-ci, avec une partie de prise (21, 31), des organes de saisie, qui maintiennent entre eux les plaques de fermeture, et une partie de reprise de forces pour exercer une force d'insertion sur l'endoprothèse intervertébrale (9), **caractérisé en ce que** les organes de saisie sont guidés en mouvement réciproque l'un vers l'autre au moyen d'une articulation (4) et peuvent être serrés sur l'endoprothèse intervertébrale (9), des saillies (51, 52) ou des cavités orientées dans la direction de serrage (12) sont formées sur les organes de saisie pour un maintien par complémentarité de forme de l'endoprothèse intervertébrale (9), et il est prévu un bloc (61) avec une face de butée (62) guidé dans la direction de l'axe longitudinal (10), qui est déplaçable au moyen d'un dispositif d'actionnement (7) pour s'appliquer sur l'endoprothèse intervertébrale (9) et qui, dans sa position avancée, immobilise l'endoprothèse intervertébrale (9) contre les saillies (51, 52) ou les cavités.

2. Instrument d'insertion selon la revendication 1, **caractérisé en ce que** l'instrument d'insertion est réalisé sous la forme d'une pince (1), dont les parties de mâchoire (22, 32) constituent les parties de saisie.

3. Instrument d'insertion selon la revendication 1 ou 2, **caractérisé en ce que** le dispositif d'actionnement (7) est une tige (71) avec une manette (72) disposée dans la région arrière de la partie de prise (21).

4. Instrument d'insertion selon la revendication 3, **caractérisé en ce que** la tige (71) est pourvue d'un filet de vis (73) et est guidée dans un filet conjugué solidaire de l'instrument, de préférence disposé dans l'articulation (4).

5. Instrument d'insertion selon la revendication 3 ou 4, **caractérisé en ce que** la manette (72) est formée par une tête à enfoncer (76).

6. Instrument d'insertion selon l'une quelconque des revendications 2 à 5, **caractérisé en ce que** le dispositif d'actionnement (7) est guidé à travers l'articulation (4).

7. Instrument d'insertion selon l'une quelconque des revendications 1 à 6, **caractérisé en ce qu'**il est prévu un dispositif d'encliquetage (8) pour fixer les parties de prise (21, 31) en position serrée, et qui présente un guidage (85) pour le dispositif d'actionnement (7).

8. Instrument d'insertion selon l'une quelconque des revendications précédentes, **caractérisé en ce que** les saillies (51, 52) sont disposées sur des inserts de mâchoire (53), qui sont fixés de manière interchangeable sur les parties de mâchoire (22, 32).
